# EUROPEAN PATENT SPECIFICATION

(11) **EP 4 403 922 B1**
(45) Date of publication and mention of the grant of the patent: **08.07.2026**
(21) Application number: 23214533.4
(22) Date of filing: 06.12.2023
(51) Int. Cl.: G01N 35/02, C12Q 1/68, C12N 15/10

(54) **TO-BE-TESTED OBJECT PREPARATION APPARATUS AND NUCLEIC ACID TESTING INTEGRATED MACHINE WITH TO-BE-TESTED OBJECT PREPARATION APPARATUS**
VORRICHTUNG ZUR HERSTELLUNG EINES ZU UNTERSUCHENDEN OBJEKTS UND INTEGRIERTE NUKLEINSÄURETESTMASCHINE MIT VORRICHTUNG ZUR HERSTELLUNG EINES ZU UNTERSUCHENDEN OBJEKTS
APPAREIL DE PRÉPARATION D'OBJET À TESTER ET MACHINE INTÉGRÉE DE TEST D'ACIDE NUCLÉIQUE AVEC APPAREIL DE PRÉPARATION D'OBJET À TESTER

(30) Priority: 19.01.2023 CN 202310090475
(43) Date of publication of application: 24.07.2024
(73) Proprietor: Shenzhen New Industries Biomedical Engineering Co., Ltd., Shenzhen, Guangdong 518122 (CN)
(72) Inventor: RAO, Jie, Shenzhen, 518122 (CN); YIN, Li, Shenzhen, 518122 (CN); TANG, Junhui, Shenzhen, 518122 (CN); ZHU, Liang, Shenzhen, 518122 (CN); WANG, Yixian, Shenzhen, 518122 (CN); ZHENG, Xiaolin, Shenzhen, 518122 (CN); HE, Guoyao, Shenzhen, 518122 (CN); XIAO, Hao, Shenzhen, 518122 (CN)
(74) Representative: Studio Torta S.p.A.

(56) References cited:
- WO-A1-2022/011604
- CN-A- 108 998 444
- CN-A- 111 621 418
- US-A1- 2019 369 134
- US-A1- 2022 062 894

## Description

### Technical Field

The invention relates to a technical field of biological detection, in particular to a to-be-tested object preparation apparatus and a nucleic acid testing integrated machine with the to-be-tested object preparation apparatus.

### Background

At present, nucleic acid testing is widely used in clinical diagnosis, agricultural monitoring, food safety, and other fields. Polymerase Chain Reaction (PCR) is the most commonly used method for nucleic acid testing. The general principle of PCR detection is a process of in vitro replication of daughter strand Deoxyribo Nucleic Acid (DNA) complementary to parent strand DNA with the parent strand DNA as a template and a specific primer as an extension starting point under the catalysis of DNA polymerase.

In the related art, when extracting and replicating the parent strand DNA, a nucleic acid testing integrated machine requires the cooperation of a plurality of modules therein, so as to extract the parent strand DNA from a sample by using an extraction reagent, and then transfer the parent strand DNA to an amplification reagent for replication.

However, the cooperation of the plurality of modules easily leads to the problem that the occupied area of the nucleic acid testing integrated machine is too large.

Examples of a to-be-tested object extraction apparatus and a nucleic acid testing integrated machine with the to-be-tested object preparation apparatus according to the prior art are known from US20190369134A1.

### Summary

The invention provides a to-be-tested object extraction apparatus and a nucleic acid testing integrated machine with the to-be-tested object preparation apparatus, so as to solve the problem that the occupied area of the nucleic acid testing integrated machine in the related technology is too large.

The invention is as defined in claim 1.

According to one aspect of the invention, a to-be-tested object extraction apparatus is provided. The to-be-tested object extraction apparatus includes: a to-be-tested object loading area, provided with a to-be-tested object loading portion, the to-be-tested object loading portion being configured to store and process a to-be-tested object tube; a sample reaction area, provided with a sample reaction portion, the sample reaction portion being configured to extract nucleic acid from a to-be-tested object; a sample adding area, provided with a sample adding portion, the sample adding portion being configured to transfer the to-be-tested object from the to-be-tested object loading portion to a first reaction vessel and add the extracted nucleic acid in the sample reaction portion to a second reaction vessel; and a grabbing area, provided with a grabbing portion, the grabbing portion being configured to move the first reaction vessel after adding the to-be-tested object from the sample adding portion to the sample reaction portion and move the first reaction vessel after extracting the nucleic acid from the to-be-tested object from the sample reaction portion to the sample adding portion. The to-be-tested object loading area, the sample adding area, and the grabbing area are arranged around the outside of the sample reaction area, and the grabbing area is located on the same side of the to-be-tested object loading area, the sample reaction area, and the sample adding area.

The to-be-tested object extraction apparatus further includes a to-be-tested object tube transfer piece, a reaction vessel transfer piece, and an extraction mechanism transfer piece. The to-be-tested object tube transfer piece is movably arranged between the to-be-tested object loading area and the sample adding area to move the to-be-tested object tube in the to-be-tested object loading area to the sample adding area, the reaction vessel transfer piece is movably arranged between the sample adding area and the grabbing area to move the first reaction vessel and the second reaction vessel in the sample adding area between the sample adding area and the grabbing area, and the extraction mechanism transfer piece is movably arranged between the sample reaction area and the grabbing area to move the first reaction vessel between the sample reaction area and the grabbing area.

In some embodiments, the to-be-tested object tube transfer piece is movably arranged between the to-be-tested object loading area and the sample adding area in a first horizontal direction, the reaction vessel transfer piece is movably arranged between the sample adding area and the grabbing area in a second horizontal direction, the extraction mechanism transfer piece is movably arranged between the sample reaction area and the grabbing area in the second horizontal direction, and the first horizontal direction is perpendicular to the second horizontal direction.

In some embodiments, the reaction vessel transfer piece includes a first reaction vessel transfer piece and a second reaction vessel transfer piece which are arranged in parallel. The first reaction vessel transfer piece and the second reaction vessel transfer piece are movably arranged between the sample adding area and the grabbing area in the second horizontal direction. The sample adding portion includes a pipetting needle. The pipetting needle is movably arranged above the first reaction vessel transfer piece and the second reaction vessel transfer piece, the first reaction vessel transfer piece is provided with a sample adding position located in the sample adding area, and when the first reaction vessel transfer piece is located in the sample adding position, the pipetting needle is able to move to above the first reaction vessel transfer piece to fill the to-be-tested object into the first reaction vessel. The second reaction vessel transfer piece is provided with a nucleic acid adding position located in the sample adding area and flush with the first reaction vessel transfer piece, and when the first reaction vessel transfer piece is located in the sample adding position and the second reaction vessel transfer piece is located in the nucleic acid adding position, the pipetting needle is able to move to above the first reaction vessel transfer piece to suck the extracted nucleic acid and move to above the second reaction vessel transfer piece to transfer the extracted nucleic acid to the second reaction vessel.

In some embodiments, the to-be-tested object tube transfer piece is provided with a second working position located in the sample adding area, and when the to-be-tested object tube transfer piece is located in the second working position, the pipetting needle is able to move to above the to-be-tested object tube transfer piece to suck the to-be-tested object.

The to-be-tested object loading portion includes a to-be-tested object tube gripper and a to-be-tested object bin configured to store the to-be-tested object tube. The to-be-tested object tube gripper is movably arranged above the to-be-tested object bin and the to-be-tested object tube transfer piece, the to-be-tested object tube transfer piece is further provided with a first working position located in the to-be-tested object loading area, and when the to-be-tested object tube transfer piece is located in the first working position, the to-be-tested object tube gripper is able to move the to-be-tested object tube from the to-be-tested object bin to the to-be-tested object tube transfer piece. The grabbing portion includes a reaction vessel gripper movably arranged in the first horizontal direction and a vertical direction. The extraction mechanism transfer piece is provided with a receiving position located in the grabbing area and an extraction position located in the sample reaction area, the reaction vessel transfer piece is provided with a first transfer position located in the grabbing area, and when the reaction vessel transfer piece is located in the first transfer position and the extraction mechanism transfer piece is located in the receiving position, the reaction vessel gripper is able to grab the first reaction vessel to move the first reaction vessel between the reaction vessel transfer piece and the extraction mechanism transfer piece.

In some embodiments, the sample adding portion further includes a consumable storage bin configured to carry a pipette tip. The pipetting needle is able to move to above the consumable storage bin to load the pipette tip.

In some embodiments, the grabbing area is further provided with a film sealing mechanism configured to encapsulate the second reaction vessel, the reaction vessel transfer piece is further provided with a second transfer position located in the grabbing area, and when the reaction vessel transfer piece is located in the second transfer position, the reaction vessel gripper is able to move to above the reaction vessel transfer piece to grab the second reaction vessel loaded with the nucleic acid and move to above the film sealing mechanism to release the second reaction vessel to the film sealing mechanism.

In some embodiments, the film sealing mechanism includes a hot pressing piece and a film sealing mechanism transfer piece. The film sealing mechanism transfer piece is movably arranged below the hot pressing piece in the second horizontal direction, the film sealing mechanism transfer piece is provided with a cover body carrying part configured to place a cover body and a second reaction vessel carrying part configured to place the second reaction vessel, the reaction vessel gripper is able to release the second reaction vessel to the second reaction vessel carrying part, the reaction vessel gripper is provided with a grabbing position located above the film sealing mechanism transfer piece, and when the reaction vessel gripper is located in the grabbing position, the film sealing mechanism transfer piece is provided with a separation position where the cover body carrying part moves to below the reaction vessel gripper, so that the reaction vessel gripper grabs the cover body, a release position where the second reaction vessel carrying part moves to below the reaction vessel gripper, so that the reaction vessel gripper releases the cover body the second reaction vessel and an encapsulation position where the second reaction vessel carrying part moves to below the hot pressing piece.

In some embodiments, the grabbing area is further provided with a waste liquid station located below the reaction vessel gripper. The waste liquid station includes a waste liquid rack, a pallet, and a waste liquid recycling piece. The liquid waste recycling piece is movably arranged on the liquid waste rack in the vertical direction and the second horizontal direction, when the reaction vessel transfer piece is located in the first transfer position, the reaction vessel gripper is able to move to above the first reaction vessel transfer piece to grab the first reaction vessel and move to above the pallet to release the first reaction vessel to the pallet, and the waste liquid recycling piece is able to move to above the pallet to recycle a magnetic rod sleeve and waste liquid.

In some embodiments, the waste liquid station further includes a support plate, a push rod, and a recycling bin. The recycling bin is located below the waste liquid rack, the pallet is vertically movably arranged on the waste liquid rack, the push rod is movably arranged on the support plate in the second horizontal direction, the support plate is provided with an avoidance opening configured to avoid the pallet, the pallet is able to move to below the support plate to remain the first reaction vessel on the support plate, and the push rod is able to push the first reaction vessel to recycle the first reaction vessel to the recycling bin.

According to another aspect of the invention, a nucleic acid testing integrated machine is provided. The nucleic acid testing integrated machine includes a reagent preparation apparatus, a to-be-tested object extraction apparatus, and an amplification apparatus. The reagent preparation apparatus is configured to prepare an extraction reagent and an amplification reagent, the amplification apparatus is configured to perform analyte detection determination on a determination mixture to analyze a to-be-tested object, and the to-be-tested object extraction apparatus is the above provided to-be-tested object extraction apparatus.

In some embodiments, the reagent preparation apparatus, the to-be-tested object extraction apparatus, and the amplification apparatus are isolated from each other, the reagent preparation apparatus and the amplification apparatus are respectively located on both sides of the to-be-tested object extraction apparatus, a first channel is arranged between the reagent preparation apparatus and the to-be-tested object extraction apparatus, a second channel is arranged between the to-be-tested object extraction apparatus and the amplification apparatus, and the first channel and the second channel have an on state and an off state. And/or, the nucleic acid testing integrated machine further includes a ferry mechanism arranged between the reagent preparation device and the to-be-tested object extraction apparatus. The ferry mechanism is able to move a first reaction vessel configured to prepare the extraction reagent and a second reaction vessel configured to prepare the amplification reagent to the to-be-tested object extraction apparatus through the first channel, and a grabbing portion of the to-be-tested object extraction apparatus is able to move the first reaction vessel to a first reaction vessel placement position of the to-be-tested object extraction apparatus and move the second reaction vessel to a second reaction vessel placement position of the to-be-tested object extraction apparatus.

By applying the technical solutions of the invention, the to-be-tested object extraction apparatus includes the to-be-tested object loading area, the sample reaction area, the sample adding area, and the grabbing area, the to-be-tested object loading area is provided with the to-be-tested object loading portion to load and process the to-be-tested object tube for containing the to-be-tested object, the sample adding area is provided with the sample adding portion to transfer the to-be-tested object from the to-be-tested object loading portion to the first reaction vessel, the grabbing area is provided with the grabbing portion to move the first reaction vessel after adding the to-be-tested object from the sample adding portion to the sample reaction portion of the sample reaction area, the nucleic acid is extracted from the to-be-tested object by using the sample reaction portion, and then the grabbing portion moves the first reaction vessel after extracting the nucleic acid from the sample reaction portion to the sample adding portion, so as to add the extracted nucleic acid in the sample reaction portion to the second reaction vessel, thereby replicating the nucleic acid. Because the to-be-tested object loading area, the sample adding area, and the grabbing area are arranged around the outside of the sample reaction area, and the grabbing area is located on the same side of the to-be-tested object loading area, the sample reaction area, and the sample adding area. Therefore, the arrangement between modules of the to-be-tested object extraction apparatus is more compact, and the arrangement of the nucleic acid testing integrated machine is also more compact, so that the occupied area of the nucleic acid testing integrated machine is reduced.

### Brief Description of the Drawings

The accompanying drawings of the specification, which constitute a part of the invention, are intended to provide a further understanding of the invention, and the exemplary embodiments of the invention and the description thereof are intended to explain the invention and do not constitute an undue limitation on the invention. In the accompanying drawings:
Fig. 1 shows a zone diagram of a to-be-tested object extraction apparatus according to an embodiment of the invention.
Fig. 2 shows a schematic structural diagram of a to-be-tested object extraction apparatus according to an embodiment of the invention.
Fig. 3 shows a schematic structural diagram of a sample reaction portion and an extraction mechanism transfer piece of a to-be-tested object extraction apparatus according to an embodiment of the invention.
Fig. 4 shows a schematic structural diagram of a film sealing mechanism of a to-be-tested object extraction apparatus according to an embodiment of the invention.
Fig. 5 shows a schematic structural diagram of a cover opening piece and a to-be-tested object tube transfer piece of a to-be-tested object extraction apparatus according to an embodiment of the invention.
Fig. 6 shows a schematic structural diagram of a waste liquid station in Fig. 2.
Fig. 7 shows a schematic structural diagram of a to-be-tested object tube transfer piece in Fig. 1.

Herein, the above accompanying drawings include the following reference signs:
10. To-be-tested object loading area; 11. To-be-tested object loading portion; 111. To-be-tested object tube gripper; 112. To-be-tested object bin; 12. Cover opening piece;
20. Sample reaction area; 21. Sample reaction portion;
30. Sample adding area; 31. Sample adding portion; 311. Pipetting needle; 312. Consumable storage bin;
40. Grabbing area; 41. Grabbing portion; 411. Reaction vessel gripper; 42. Waste liquid station; 421. Waste liquid rack; 422. Pallet; 423. Waste liquid recycling piece; 424. Support plate; 425. Push rod; 43. Film sealing mechanism; 431. Hot pressing piece; 432. Film sealing mechanism transfer pieces; 433. Cover body carrying part; 434. Second reaction vessel carrying part;
50. To-be-tested object tube transfer piece;
60. First reaction vessel transfer piece;
70. Second reaction vessel transfer piece;
80. Extraction mechanism transfer piece.

### Detailed Description of the Embodiments

The technical solutions in the embodiments of the invention will be clearly and completely described in conjunction with the accompanying drawings in the embodiments of the invention. It is apparent that the described embodiments are only a part of the embodiments of the invention, and not all of them. The following description of at least one exemplary embodiment is only illustrative, and in no way serves as any limitation on the invention or application or use thereof.

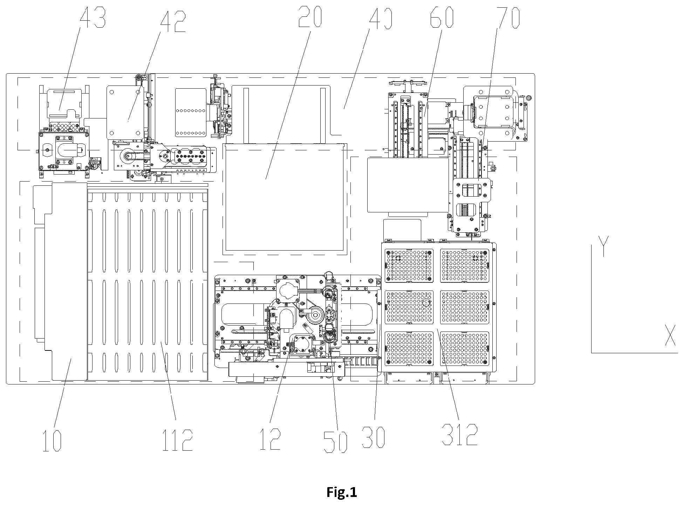

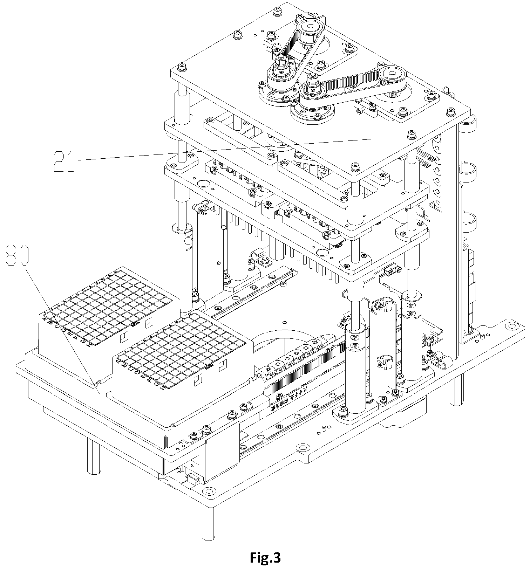

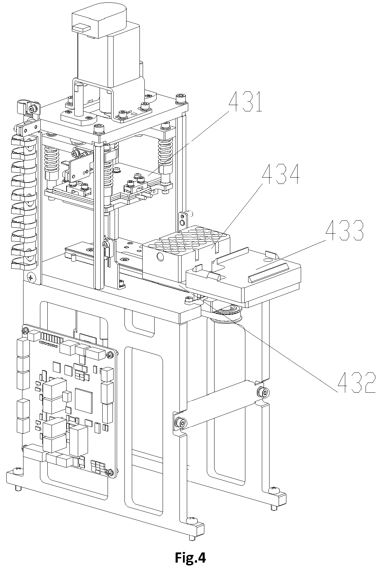

As shown in Fig. 1 to Fig. 6, the embodiments of the invention provide a to-be-tested object extraction apparatus. The to-be-tested object extraction apparatus includes: a to-be-tested object loading area 10, a sample reaction area 20, a sample adding area 30, and a grabbing area 40. The to-be-tested object loading area 10 is provided with a to-be-tested object loading portion 11, the to-be-tested object loading portion 11 being configured to store and process a to-be-tested object tube. The sample reaction area 20 is provided with a sample reaction portion 21, the sample reaction portion 21 being configured to extract nucleic acid from the to-be-tested object. The sample adding area 30 is provided with a sample adding portion 31, the sample adding portion 31 being configured to transfer the to-be-tested object from the to-be-tested object loading portion 11 to a first reaction vessel and add the extracted nucleic acid in the sample reaction portion 21 to a second reaction vessel. The grabbing area 40 is provided with a grabbing portion 41, the grabbing portion 41 being configured to move the first reaction vessel after adding the to-be-tested object from the sample adding portion 31 to the sample reaction portion 21 and move the extracted nucleic acid in the to-be-tested object from the sample reaction portion 21 to the sample adding portion 31. The to-be-tested object loading area 10, the sample adding area 30, and the grabbing area 40 are arranged around the outside of the sample reaction area 20, and the grabbing area 40 is located on the same side of the to-be-tested object loading area 10, the sample reaction area 20, and the sample adding area 30.

By applying the technical solutions of the invention, the to-be-tested object extraction apparatus includes the to-be-tested object loading area 10, the sample reaction area 20, the sample adding area 30, and the grabbing area 40, the to-be-tested object loading area 10 is provided with the to-be-tested object loading portion 11 to load and process the to-be-tested object tube for containing the to-be-tested object, the sample adding area 30 is provided with the sample adding portion 31 to transfer the to-be-tested object from the to-be-tested object loading portion 11 to the first reaction vessel, the grabbing area 40 is provided with the grabbing portion 41 to move the first reaction vessel after adding the to-be-tested object from the sample adding portion 31 to the sample reaction portion 21 of the sample reaction area 20, the nucleic acid is extracted from the to-be-tested object by using the sample reaction portion 21, and then the grabbing portion 41 moves the first reaction vessel after extracting the nucleic acid from the sample reaction portion 21 to the sample adding portion 31, so as to add the extracted nucleic acid in the sample reaction portion 21 to the second reaction vessel, thereby replicating the nucleic acid. Because the to-be-tested object loading area 10, the sample adding area 30, and the grabbing area 40 are arranged around the outside of the sample reaction area 20, and the grabbing area 40 is located on the same side of the to-be-tested object loading area 10, the sample reaction area 20, and the sample adding area 30. Therefore, the arrangement between modules of the to-be-tested object extraction apparatus is more compact, and the arrangement of the nucleic acid testing integrated machine is also more compact, so that the occupied area of the nucleic acid testing integrated machine is reduced.

In the embodiment, the sample reaction portion 21 includes an extraction mechanism. The nucleic acid is extracted from a sample in the first reaction vessel by using the extraction mechanism. The extraction mechanism may be a mechanism structure in the prior art, such as the structure in the Chinese Patent Application No. 202222368037.2.

It is to be noted that a transfer mechanism is arranged between areas of the to-be-tested object extraction apparatus to realize the transfer of consumables and the to-be-tested object between the areas.

As shown in Fig. 1 to Fig. 3 and Fig. 5, the to-be-tested object extraction apparatus further includes a to-be-tested object tube transfer piece 50, a reaction vessel transfer piece, and an extraction mechanism transfer piece 80. The to-be-tested object tube transfer piece 50 is movably arranged between the to-be-tested object loading area 10 and the sample adding area 30 to move the to-be-tested object tube in the to-be-tested object loading area 10 to the sample adding area 30, the reaction vessel transfer piece is movably arranged between the sample adding area 30 and the grabbing area 40 to move the first reaction vessel and the second reaction vessel in the sample adding area 30 between the sample adding area 30 and the grabbing area 40, and the extraction mechanism transfer piece 80 is movably arranged between the sample reaction area 20 and the grabbing area 40 to move the first reaction vessel between the sample reaction area 20 and the grabbing area 40. The to-be-tested object tube transfer piece 50 is able to move the to-be-tested object tube from the to-be-tested object loading area 10 to the sample adding area 30, so that the to-be-tested object is transferred to the sample adding area 30 to be transferred to the first reaction vessel. The reaction vessel transfer piece is able to move the first reaction vessel between the sample adding area 30 and the grabbing area 40, so as to move the first reaction vessel carrying the to-be-tested object from the sample adding area 30 to the grabbing area 40. The extraction mechanism transfer piece 80 moves the first reaction vessel carrying the to-be-tested object from the grabbing area 40 to the sample reaction area 20. The nucleic acid is extracted from the to-be-tested object by using the sample reaction portion 21. The extraction mechanism transfer piece 80 moves the first reaction vessel after extracting the nucleic acid from the sample reaction area 20 to the grabbing area 40, and then the reaction vessel transfer piece moves the first reaction vessel after extracting the nucleic acid from the grabbing area 40 to the sample adding area 30, and transfers the extracted nucleic acid from the first reaction vessel to the second reaction vessel.

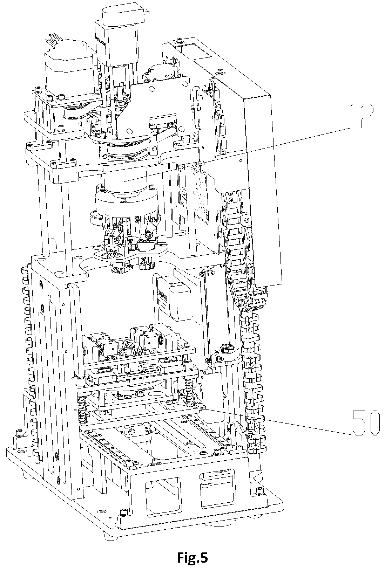

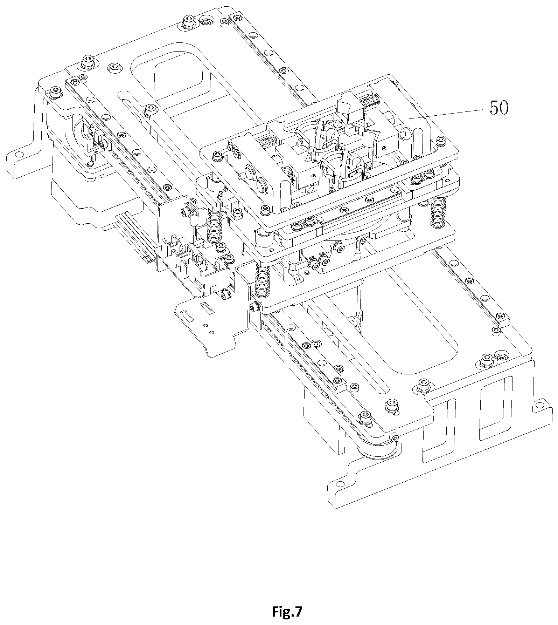

As shown in Fig. 1 and Fig. 5, the to-be-tested object tube transfer piece 50 is movably arranged between the to-be-tested object loading area 10 and the sample adding area 30 in a first horizontal direction by a first driving mechanism. As shown in Fig. 7, the to-be-tested object tube transfer piece 50 may be a first base with a first carrying part for carrying the to-be-tested object tube. The first driving mechanism may be a belt mechanism or a cylinder mechanism.

The reaction vessel transfer piece is movably arranged between the sample adding area 30 and the grabbing area 40 in a second horizontal direction, the extraction mechanism transfer piece 80 is movably arranged between the sample reaction area 20 and the grabbing area 40 in the second horizontal direction by a second driving mechanism, and the first horizontal direction is perpendicular to the second horizontal direction. The extraction mechanism transfer piece 80 may be a second base with a second carrying part for carrying the first reaction vessel. The second driving mechanism may be a belt mechanism or a cylinder mechanism.

The to-be-tested object tube transfer piece 50 moves in the first horizontal direction, and the reaction vessel transfer piece and the extraction mechanism transfer piece 80 move in the second horizontal direction, which shortens moving paths of the to-be-tested object tube transfer piece 50, the reaction vessel transfer piece, and the extraction mechanism transfer piece 80, and then reduces the space occupation, so as to reduce the occupied area of the nucleic acid integrated machine.

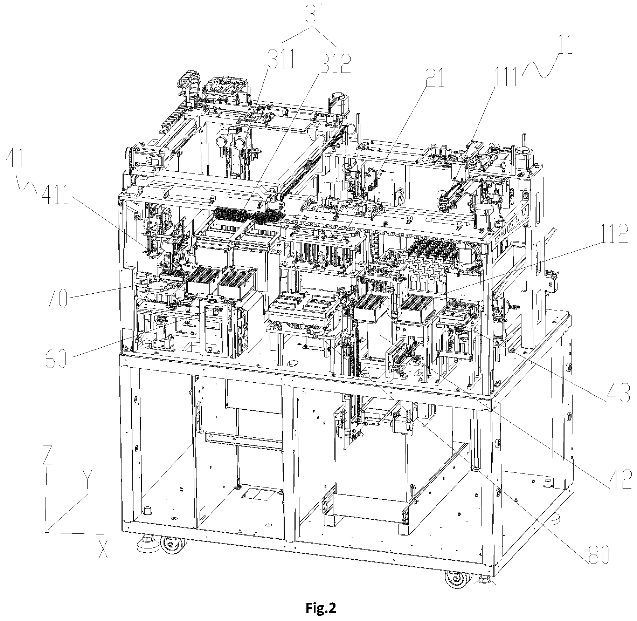

As shown in Fig. 1 and Fig. 2, the reaction vessel transfer piece includes a first reaction vessel transfer piece 60 and a second reaction vessel transfer piece 70 which are arranged in parallel. The first reaction vessel transfer piece 60 and the second reaction vessel transfer piece 70 are movably arranged between the sample adding area 30 and the grabbing area 40 in the second horizontal direction. The structure and the driving mode of the first reaction vessel transfer piece 60 and the second reaction vessel transfer piece 70 are similar to that of the extraction mechanism transfer piece 80.

The sample adding portion 31 includes a pipetting needle 311. The pipetting needle 311 is movably arranged above the first reaction vessel transfer piece 60 and the second reaction vessel transfer piece 70, the first reaction vessel transfer piece 60 is provided with a sample adding position located in the sample adding area 30, and when the first reaction vessel transfer piece 60 is located in the sample adding position, the pipetting needle 311 is able to move to above the first reaction vessel transfer piece 60 to fill the to-be-tested object into the first reaction vessel. The pipetting needle 311 is able to suck the to-be-tested object in the to-be-tested object tube and release it into the first reaction vessel on the first reaction vessel transfer piece 60, and the first reaction vessel transfer piece 60 moves the first reaction vessel filled with the to-be-tested object from the sample adding area 30 to the grabbing area 40.

In the embodiment, the to-be-tested object extraction apparatus is provided with a frame body, the to-be-tested object loading portion 11, the sample reaction portion 21, the sample adding portion 31, and the grabbing portion 41 are arranged in the frame body, and the sample adding portion 31 further includes a sample arm. The sample arm is movably arranged on the frame body in the horizontal direction, and the pipetting needle 311 is movably arranged on the sample arm in the vertical direction, so that the three-axis movement of the pipetting needle 311 is realized.

Specifically, the second reaction vessel transfer piece 70 is provided with a nucleic acid adding position located in the sample adding area 30 and flush with the first reaction vessel transfer piece 60, and when the first reaction vessel transfer piece 60 is located in the sample adding position and the second reaction vessel transfer piece 70 is located in the nucleic acid adding position, the pipetting needle 311 is able to move to above the first reaction vessel transfer piece 60 to suck the extracted nucleic acid and move to above the second reaction vessel transfer piece 70 to transfer the extracted nucleic acid to the second reaction vessel. The extracted nucleic acid in the sample reaction portion 21 is transferred to the second reaction vessel located in the second reaction vessel transfer piece 70 by using the pipetting needle 311, and then the nucleic acid is replicated by using an amplification reagent in the second reaction vessel.

The to-be-tested object tube transfer piece 50 is provided with a second working position located in the sample adding area 30, and when the to-be-tested object tube transfer piece 50 is located in the second working position, the pipetting needle 311 is able to move to above the to-be-tested object tube transfer piece 50 to suck the to-be-tested object. The to-be-tested object tube transfer piece 50 moves the to-be-tested object tube from the to-be-tested object loading area 10 to the sample adding area 30, and the pipetting needle 311 sucks the to-be-tested object in the to-be-tested object tube on the to-be-tested object tube transfer piece 50 and fill it to the first reaction vessel on the first reaction vessel transfer piece 60.

In the embodiment, through the arrangement of the reaction vessel transfer piece, a sample adding function of filling the to-be-tested object from the to-be-tested object tube to the first reaction vessel and a nucleic acid transfer function of filling the extracted nucleic acid from the first reaction vessel to the second reaction vessel are realized in the sample adding area 30, which is conducive to the simplification of the structure of the to-be-tested object extraction apparatus.

As shown in Fig. 2, the to-be-tested object loading portion 11 includes a to-be-tested object tube gripper 111 and a to-be-tested object bin 112 configured to store the to-be-tested object tube. The to-be-tested object tube gripper 111 is movably arranged above the to-be-tested object bin 112 and the to-be-tested object tube transfer piece 50, the to-be-tested object tube transfer piece 50 is further provided with a first working position located in the to-be-tested object loading area 10, and when the to-be-tested object tube transfer piece 50 is located in the first working position, the to-be-tested object tube gripper 111 is able to move the to-be-tested object tube from the to-be-tested object bin 112 to the to-be-tested object tube transfer piece 50. The to-be-tested object tube gripper 111 is able to move the to-be-tested object tube in the to-be-tested object bin 112 to the to-be-tested object tube transfer piece 50, and then the movement of the to-be-tested object tube between the to-be-tested object loading area 10 and the sample adding area 30 is realized by using the to-be-tested object tube transfer piece 50.

In the embodiment, the to-be-tested object tube gripper 111 realizes the three-axis movement on the frame body, so as to move the to-be-tested object tube from the to-be-tested object bin 112 to the to-be-tested object tube transfer piece 50. The frame body is provided with a transverse guide rail and a longitudinal guide rail. The to-be-tested object tube gripper 111 includes a gripper frame and a clamping jaw vertically movably arranged on the gripper frame. The gripper frame is able to move in the extension direction of the transverse guide rail and the longitudinal guide rail to realize the movement of the gripper frame in the horizontal plane, and then realize the three-axis movement of the clamping jaw.

It is to be noted that the to-be-tested object includes samples, quality control products, calibration products, etc.

As shown in Fig. 1 and Fig. 2, the grabbing portion 41 includes a reaction vessel gripper 411 movably arranged in the first horizontal direction and a vertical direction. The extraction mechanism transfer piece 80 is provided with a receiving position located in the grabbing area 40 and an extraction position located in the sample reaction area 20, the reaction vessel transfer piece is provided with a first transfer position located in the grabbing area 40, and when the reaction vessel transfer piece is located in the first transfer position and the extraction mechanism transfer piece 80 is located in the receiving position, the reaction vessel gripper 411 is able to grab the first reaction vessel to move the first reaction vessel between the reaction vessel transfer piece and the extraction mechanism transfer piece 80. The reaction vessel gripper 411 is able to move the first reaction vessel carrying the to-be-tested object from the reaction vessel transfer piece to the extraction mechanism transfer piece 80, and the extraction mechanism transfer piece 80 moves from a grabbing position to an extraction position to move the first reaction vessel to the extraction mechanism, so as to extract the nucleic acid from the to-be-tested object. In addition, after extracting the nucleic acid, the extraction mechanism transfer piece 80 is able to move the first reaction vessel after extracting the nucleic acid from the extraction position to the grabbing position, and then the reaction vessel gripper 411 moves the first reaction vessel from the extraction mechanism transfer piece 80 to the reaction vessel transfer piece.

In the embodiment, the first reaction vessel transfer piece 60 is located in the first transfer position, and the reaction vessel gripper 411 grabs the first reaction vessel to move the first reaction vessel between the first reaction vessel transfer piece 60 and the extraction mechanism transfer piece 80.

In the embodiment, the transmission of consumables between the areas is realized through the coordination of the movement of the transfer piece between the areas in the horizontal direction and the movement of the gripper in the area in the horizontal direction and the vertical direction, and the areas operate independently and work in parallel, the compact structure is realized, and the work efficiency is ensured.

Specifically, the to-be-tested object loading portion 11 further includes a cover opening piece 12. The to-be-tested object tube transfer piece 50 is further provided with a third working position. When the to-be-tested object tube transfer piece 50 is located in the third working position, the to-be-tested object tube transfer piece 50 is located below the cover opening piece 12, and the third working position is located between the second working position and the first working position. A cover of the to-be-tested object tube located on the to-be-tested object tube transfer piece 50 is opened by using the cover opening piece 12, so that the sample adding portion 31 is able to suck the to-be-tested object in the to-be-tested object tube.

In the embodiment, the frame body is provided with a vessel gripper guide rail extending in the first horizontal direction, and the reaction vessel gripper 411 includes a vessel gripper frame and an orifice plate clamping jaw. The vessel gripper frame is movably arranged on the vessel gripper guide rail in the extension direction of the vessel gripper guide rail, and the orifice plate clamping jaw is vertically movably arranged on the vessel gripper frame. Through the adoption of the above structure, the two-axis movement of the reaction vessel gripper 411 is realized.

Specifically, both the first reaction vessel transfer piece 60 and the extraction mechanism transfer piece 80 are able to move to the grabbing area 40, and then the reaction vessel gripper 411 is able to move the first reaction vessel filled with the to-be-tested object from the first reaction vessel transfer piece 60 to the extraction mechanism transfer piece 80. Then, the extraction mechanism transfer piece 80 moves the first reaction vessel to the extraction position, so that the nucleic acid is extracted by using the extraction mechanism.

As shown in Fig. 2, the sample adding portion 31 further includes a consumable storage bin 312 configured to carry a pipette tip. The pipetting needle 311 is able to move to above the consumable storage bin 312 to load the pipette tip. The pipette tip is stored by using the consumable storage bin 312, and then the pipette tip is loaded by using the pipetting needle 311.

Specifically, the pipetting needle 311 needs to move to above the consumable storage bin 312 to load the pipette tip before the pipetting needle 311 sucks the to-be-tested object in the to-be-tested object tube. Then, the to-be-tested object is sucked by using the pipette tip. The process of sucking the nucleic acid is similar to the above, and will not be elaborated herein.

In the embodiment, the grabbing area 40 is further provided with a film sealing mechanism 43, the reaction vessel transfer piece is further provided with a second transfer position located in the grabbing area 40, and when the reaction vessel transfer piece is located in the second transfer position, the reaction vessel gripper 411 is able to move to above the reaction vessel transfer piece to grab the second reaction vessel loaded with the nucleic acid and move to above the film sealing mechanism 43 to release the second reaction vessel to the film sealing mechanism 43. The second reaction vessel loaded with the nucleic acid is transferred to the film sealing mechanism 43 by using the reaction vessel gripper 411, and the second reaction vessel loaded with the nucleic acid is encapsulated by using the film sealing mechanism 43.

Specifically, the second reaction vessel transfer piece 70 is located in the second transfer position, and the reaction vessel gripper 411 is able to move to above the second reaction vessel transfer piece 70 to grab the second reaction vessel loaded with the nucleic acid.

As shown in Fig. 1, Fig. 2 and Fig. 4, the film sealing mechanism 43 includes a hot pressing piece 431 and a film sealing mechanism transfer piece 432. The film sealing mechanism transfer piece 432 is movably arranged below the hot pressing piece 431 in the second horizontal direction, the film sealing mechanism transfer piece 432 is provided with a cover body carrying part 433 configured to place a cover body and a second reaction vessel carrying part 434 configured to place the second reaction vessel, the reaction vessel gripper 411 is able to release the second reaction vessel to the second reaction vessel carrying part 434, the reaction vessel gripper 411 is provided with a grabbing position located above the film sealing mechanism transfer piece 432, and when the reaction vessel gripper 411 is located in the grabbing position, the film sealing mechanism transfer piece 432 is provided with a separation position where the cover body carrying part 433 moves to below the reaction vessel gripper, so that the reaction vessel gripper 411 grabs the cover body, a release position where the second reaction vessel carrying part 434 moves to below the reaction vessel gripper 411, so that the reaction vessel gripper 411 releases the cover body to the second reaction vessel and an encapsulation position where the second reaction vessel carrying part 434 moves to below the hot pressing piece 431. Through the adoption of the above structure, the cover body is carried by using the cover body carrying part 433 of the film sealing mechanism transfer piece 432, and the second reaction vessel is carried by using the second reaction vessel carrying part 434.

The reaction vessel gripper 411 moves the reaction second vessel loaded with the nucleic acid to the film sealing mechanism transfer piece 432, and moves the cover body from the cover body carrying part 433 to the second reaction vessel, and then the encapsulation of the cover body and the second reaction vessel is completed by using the hot pressing piece 431.

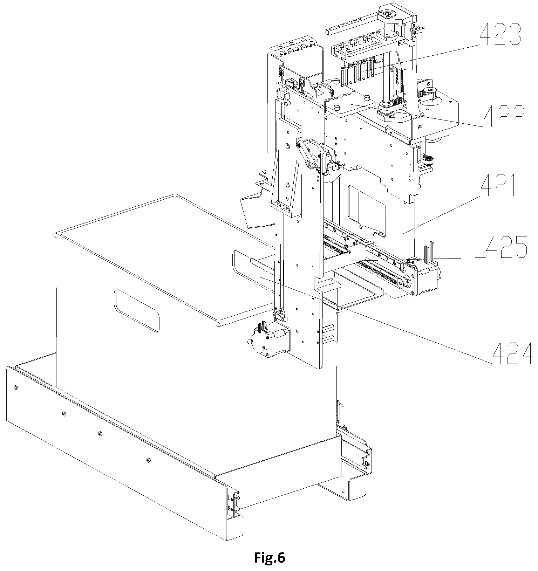

As shown in Fig. 2 and Fig. 6, the grabbing area 40 is further provided with a waste liquid station 42 located below the reaction vessel gripper 411. The waste liquid station 42 includes a waste liquid rack 421, a pallet 422, and a waste liquid recycling piece 423. The liquid waste recycling piece 423 is movably arranged on the liquid waste rack 421 in the vertical direction and the second horizontal direction, when the first reaction vessel transfer piece 60 is located in the second transfer position, the reaction vessel gripper 411 is able to move to above the first reaction vessel transfer piece 60 to grab the first reaction vessel and move to above the pallet 422 to release the first reaction vessel to the pallet 422, and the waste liquid recycling piece 423 is able to move to above the pallet 422 to recycle a magnetic rod sleeve and waste liquid. The first reaction vessel on the first reaction vessel transfer piece 60 after extracting the nucleic acid is transferred to the pallet 422 by using the reaction vessel gripper 411, and the waste liquid and the magnetic rod sleeve in the first reaction vessel are recycled by using the waste liquid recycling piece 423.

In the embodiment, an upper end of the waste liquid recycling piece 423 is provided with a protruding portion, the magnetic rod sleeve is recycled by using the protruding portion, a lower end of the waste liquid recycling piece 423 is provided with a waste liquid needle, and the waste liquid is sucked by using the waste liquid needle.

As shown in Fig. 6, the waste liquid station 42 further includes a support plate 424, a push rod 425, and a recycling bin. The recycling bin is located below the waste liquid rack 421, the pallet 422 is vertically movably arranged on the waste liquid rack 421, the push rod 425 is movably arranged on the support plate 424 in the second horizontal direction, the support plate 424 is provided with an avoidance opening configured to avoid the pallet 422, the pallet 422 is able to move to below the support plate 424 to remain the first reaction vessel on the support plate 424, and the push rod 425 is able to push the first reaction vessel to recycle the first reaction vessel to the recycling bin. The pallet 422 is avoided by using the avoidance opening of the support plate 424, and then the first reaction vessel on the pallet 422 remains on the support plate 424. The push rod 425 is able to move the first reaction vessel on the support plate 424 to the recycling bin.

Specifically, after the first reaction vessel after extracting the nucleic acid is transferred to the pallet 422 by using the reaction vessel gripper 411, the waste liquid recycling piece 423 moves to above the first reaction vessel to recycle the magnetic rod sleeve and the waste liquid. After the magnetic rod sleeve and the waste liquid are recycled, the pallet 422 vertically moves and penetrates through the avoidance opening, so that the first reaction vessel remains on the support plate 424, and then the push rod 425 is able to move the first reaction vessel on the support plate 424 to the recycling bin.

In the embodiment, when extracting the nucleic acid in the to-be-tested object, the to-be-tested object tube gripper 111 moves to above the to-be-tested object bin 112 to grab the to-be-tested object tube to the to-be-tested object tube transfer piece 50 located in the first working position, the to-be-tested object tube is transferred to the third working position below the cover opening piece 12 by the to-be-tested object tube transfer piece 50, the cover is opened by using the cover opening piece 12, and after the cover is opened, the to-be-tested object tube moves to the second working position in the sample adding area 30 by the to-be-tested object tube transfer piece 50, and the pipetting needle 311 moves to above the to-be-tested object tube transfer piece 50 to suck the to-be-tested object. Then, the pipetting needle 311 moves to the sample adding area 30, and moves to above the first reaction vessel transfer piece 60 in the sample adding position, so as to release the to-be-tested object into the first reaction vessel of the first reaction vessel transfer piece 60 by using the pipetting needle 311. Then, the first reaction vessel transfer piece 60 moves to the first transfer position located in the grabbing area 40, and the reaction vessel gripper 411 grabs the first reaction vessel on the first reaction vessel transfer piece 60. Then, the reaction vessel gripper 411 moves to above the extraction mechanism transfer piece 80 located in the receiving position, so as to release the first reaction vessel to the extraction mechanism transfer piece 80, and move the extraction mechanism transfer piece 80 to the extraction position in the extraction mechanism, so that the nucleic acid in the to-be-tested object is extracted by using the extraction mechanism. After the nucleic acid is extracted, the extraction mechanism transfer piece 80 moves from the extraction position located in the sample reaction area to the receiving position located in the grabbing area 40, and the reaction vessel gripper 411 grabs the first reaction vessel and releases it to the first reaction vessel transfer piece 60 located in the first transfer position. Then, the first reaction vessel transfer piece 60 moves to the sample adding position located in the sample adding area 30, the extracted nucleic acid in the first reaction vessel is sucked by using the pipetting needle 311, and then the pipetting needle 311 moves to above the second reaction vessel transfer piece 70 located in the nucleic acid adding position, so as to release the nucleic acid into the second reaction vessel. Then, the second reaction vessel transfer piece 70 moves to the second transfer position located in the grabbing area 40, the reaction vessel gripper 411 grabs the second reaction vessel, and the reaction vessel gripper 411 moves to the grabbing position located above the film sealing mechanism transfer piece 432 and release the second reaction vessel to the second reaction vessel carrying part 434. The film sealing mechanism transfer piece 432 moves to the separation position, the reaction vessel gripper 411 grabs the cover body, then the film sealing mechanism transfer piece 432 moves to the release position to release the cover body to the second reaction vessel, and then the film sealing mechanism transfer piece 432 moves to the encapsulation position to encapsulate the cover body and the second reaction vessel by using the hot pressing piece 431.

The first horizontal direction refers to an X-axis direction in Fig. 2, the second horizontal direction refers to a Y-axis direction in Fig. 2, and the vertical direction refers to a Z-axis direction in Fig. 2.

Another embodiment of the invention provides a nucleic acid testing integrated machine. The nucleic acid testing integrated machine includes a reagent preparation apparatus, a to-be-tested object extraction apparatus, and an amplification apparatus. The reagent preparation apparatus is configured to prepare an extraction reagent and an amplification reagent, the amplification apparatus is configured to perform analyte detection determination on a determination mixture to analyze a to-be-tested object, and the to-be-tested object extraction apparatus is the above provided to-be-tested object extraction apparatus. Through the adoption of the nucleic acid testing integrated machine, because the to-be-tested object loading area 10, the sample adding area 30, and the grabbing area 40 are arranged around the outside of the sample reaction area 20, the grabbing area 40 is located on the same side of the to-be-tested object loading area 10, the sample reaction area 20, and the sample adding area 30. Therefore, the arrangement between modules of the to-be-tested object extraction apparatus is more compact, and the arrangement of the nucleic acid testing integrated machine is also more compact.

In the embodiment, the reagent preparation apparatus, the to-be-tested object extraction apparatus, and the amplification apparatus are isolated from each other, the reagent preparation apparatus and the amplification apparatus are respectively located on both sides of the to-be-tested object extraction apparatus, a first channel is arranged between the reagent preparation apparatus and the to-be-tested object extraction apparatus, a second channel is arranged between the to-be-tested object extraction apparatus and the amplification apparatus, and the first channel and the second channel have an on state and an off state. Through the adoption of the above structure, isolation between modules of the apparatus is realized by closing the first channel and the second channel, thereby avoiding cross contamination. The transmission of consumables between the modules of the apparatus is realized by conducting the first channel and the second channel.

The nucleic acid testing integrated machine further includes a ferry mechanism arranged between the reagent preparation device and the to-be-tested object extraction apparatus. The ferry mechanism is able to move a first reaction vessel configured to prepare the extraction reagent and a second reaction vessel configured to prepare the amplification reagent to the to-be-tested object extraction apparatus through the first channel, and a grabbing portion 41 of the to-be-tested object extraction apparatus is able to move the first reaction vessel to a first reaction vessel placement position of the to-be-tested object extraction apparatus and move the second reaction vessel to a second reaction vessel placement position of the to-be-tested object extraction apparatus. The ferry mechanism moves the first reaction vessel that completes the preparation of the extraction reagent and the second reaction vessel that completes the preparation of the amplification reagent in the reagent preparation apparatus to the to-be-tested object extraction apparatus for the extraction of the to-be-tested object.

It is to be noted that the terms used herein is only for the purpose of describing the specific implementation modes and is not intended to limit the exemplary implementation modes of the invention. As used herein, the singular form is also intended to include the plural form unless otherwise expressly stated in the context, and it should also be understood that when the terms "contain" and/or "include" are used in the specification, they indicate the presence of features, steps, operations, devices, components and/or combinations thereof.

Unless otherwise specified, the relative arrangement, numerical expressions and numerical values of parts and steps set forth in these embodiments do not limit the scope of the invention. Also, it should be understood that, for ease of description, the dimensions of various parts shown in the accompanying drawings are not drawn to scale. Techniques, methods, and devices known to those of ordinary skill in the related fields may not be discussed in detail, but should be regarded as part of the specification under appropriate circumstances. In all examples shown and discussed herein, any specific value should be interpreted as exemplary only and not as a limitation. Thus, other examples of the exemplary embodiments may have different values. It is to be noted that: similar numbers and letters refer to similar items in the following accompanying drawings, and thus, once an item is defined in one accompanying drawing, it does not require further discussion in subsequent accompanying drawings.

In the description of the invention, it is to be understood that the orientations or positional relationships indicated by the orientation words "front, rear, upper, down, left and right", "transverse, longitudinal, vertical and horizontal", "top and bottom", etc. are based on the orientations or positional relationships shown in the accompanying drawings, and are only for the convenience of describing the invention and simplifying the description. Unless stated to the contrary, these orientation words do not indicate or imply that the apparatus or element referred to must have a specific orientation, be constructed and operated in a specific orientation, and therefore cannot be construed as limiting the scope of protection of the invention. The orientation words "inside and outside" refer to inside and outside relative to the outline of each part itself.

For ease of description, spatially relative terms, such as "over", "above", "on the surface", "upper", etc., may be used herein to describe the spatial positional relationship between a device or feature and other devices or features as shown in the figures. It should be understood that the spatially relative terms are intended to include different orientations of the device in use or operation in addition to the orientation described in the figures. For example, if the devices in the accompanying drawings are inverted, those described as "above other devices or structures" or "over other devices or structures" will then be positioned as "below other devices or structures" or "under other devices or structures". Thus, the exemplary term "above" may include both "above" and "below" orientations. The device may also be positioned in various other ways (rotated 90 degrees or at other orientations) and the spatially relative descriptions used herein are interpreted accordingly.

Furthermore, it is to be noted that the use of the words "first", "second" and the like to define parts is only for the convenience of distinguishing the corresponding parts, unless otherwise stated, the words have no special meaning, and therefore cannot be construed as limiting the scope of protection of the invention.

The above is only the preferred embodiments of the invention, and is not intended to limit the invention, and for those of ordinary skill in the art, various modifications and changes may be made within the scope of the appended claims.

## Claims

1. A to-be-tested object extraction apparatus, comprising:
a to-be-tested object loading area (10), provided with a to-be-tested object loading portion (11), the to-be-tested object loading portion (11) being configured to store and process a to-be-tested object tube; wherein, the to-be-tested object loading portion (11) comprises a to-be-tested object bin (112) configured to store the to-be-tested object tube;
a sample reaction area (20), provided with a sample reaction portion (21), the sample reaction portion (21) being configured to extract nucleic acid from a to-be-tested object; the sample reaction portion (21) comprising an extraction mechanism;
a sample adding area (30), provided with a sample adding portion (31), the sample adding portion (31) being configured to transfer the to-be-tested object from the to-be-tested object loading portion (11) to a first reaction vessel and add an extracted nucleic acid in the sample reaction portion (21) to a second reaction vessel;
a to-be-tested object tube transfer piece (50), which is movably arranged between the to-be-tested object loading area (10) and the sample adding area (30) to move the to-be-tested object tube in the to-be-tested object loading area (10) to the sample adding area (30), the to-be-tested object tube transfer piece (50) provided with a first working position located in the to-be-tested object loading area (10); and
a grabbing area (40), provided with a grabbing portion (41);
wherein the to-be-tested object loading area (10), the sample adding area (30), and the grabbing area (40) are arranged around an outside of the sample reaction area (20), and the grabbing area (40) is located on a same side of the to-be-tested object loading area (10), the sample reaction area (20), and the sample adding area (30);
**characterized in that**
the to-be-tested object extraction apparatus further comprises a reaction vessel transfer piece, and an extraction mechanism transfer piece (80); wherein the reaction vessel transfer piece is movably arranged between the sample adding area (30) and the grabbing area (40) to move the first reaction vessel and the second reaction vessel in the sample adding area (30) between the sample adding area (30) and the grabbing area (40), and the extraction mechanism transfer piece (80) is movably arranged between the sample reaction area (20) and the grabbing area (40) to move the first reaction vessel between the sample reaction area (20) and the grabbing area (40);
the grabbing portion (41) is configured to move the first reaction vessel after adding the to-be-tested object from the reaction vessel transfer piece to the extraction mechanism transfer piece (80) and move the first reaction vessel after extracting the nucleic acid from the to-be-tested object from the extraction mechanism transfer piece (80) to the reaction vessel transfer piece;
and the to-be-tested object loading portion (11) further comprises a to-be-tested object tube gripper (111), wherein the to-be-tested object tube gripper (111) is movably arranged above the to-be-tested object bin (112) and the to-be-tested object tube transfer piece (50), and when the to-be-tested object tube transfer piece (50) is located in the first working position, the to-be-tested object tube gripper (111) is able to move the to-be-tested object tube from the to-be-tested object bin (112) to the to-be-tested object tube transfer piece (50).

2. The to-be-tested object extraction apparatus according to claim 1, wherein the to-be-tested object tube transfer piece (50) is movably arranged between the to-be-tested object loading area (10) and the sample adding area (30) in a first horizontal direction, the reaction vessel transfer piece is movably arranged between the sample adding area (30) and the grabbing area (40) in a second horizontal direction, the extraction mechanism transfer piece (80) is movably arranged between the sample reaction area (20) and the grabbing area (40) in the second horizontal direction, and the first horizontal direction is perpendicular to the second horizontal direction.

3. The to-be-tested object extraction apparatus according to claim 2, wherein,
the reaction vessel transfer piece comprises a first reaction vessel transfer piece (60) and a second reaction vessel transfer piece (70) which are arranged in parallel, wherein the first reaction vessel transfer piece (60) and the second reaction vessel transfer piece (70) are movably arranged between the sample adding area and the grabbing area in the second horizontal direction; the sample adding portion (31) comprises a pipetting needle (311), wherein the pipetting needle (311) is movably arranged above the first reaction vessel transfer piece (60) and the second reaction vessel transfer piece (70), the first reaction vessel transfer piece (60) is provided with a sample adding position located in the sample adding area (30), and when the first reaction vessel transfer piece (60) is located in the sample adding position, the pipetting needle (311) is able to move to above the first reaction vessel transfer piece (60) to fill the to-be-tested object into the first reaction vessel.

4. The to-be-tested object extraction apparatus according to claim 3, wherein,
the second reaction vessel transfer piece (70) is provided with a nucleic acid adding position located in the sample adding area (30) and flush with the first reaction vessel transfer piece (60), and when the first reaction vessel transfer piece (60) is located in the sample adding position and the second reaction vessel transfer piece (70) is located in the nucleic acid adding position, the pipetting needle (311) is able to move to above the first reaction vessel transfer piece (60) to suck the extracted nucleic acid and move to above the second reaction vessel transfer piece (70) to transfer the extracted nucleic acid to the second reaction vessel.

5. The to-be-tested object extraction apparatus according to claim 3, wherein the to-be-tested object tube transfer piece (50) is provided with a second working position located in the sample adding area (30), and when the to-be-tested object tube transfer piece (50) is located in the second working position, the pipetting needle (311) is able to move to above the to-be-tested object tube transfer piece (50) to suck the to-be-tested object.

6. The to-be-tested object extraction apparatus according to claim 3, wherein,
the grabbing portion (41) comprises a reaction vessel gripper (411) movably arranged in the first horizontal direction and a vertical direction, wherein the extraction mechanism transfer piece (80) is provided with a receiving position located in the grabbing area (40) and an extraction position located in the sample reaction area (20), the reaction vessel transfer piece is provided with a first transfer position located in the grabbing area (40), and when the reaction vessel transfer piece is located in the first transfer position and the extraction mechanism transfer piece (80) is located in the receiving position, the reaction vessel gripper (411) is able to grab the first reaction vessel to move the first reaction vessel between the reaction vessel transfer piece and the extraction mechanism transfer piece (80).

7. The to-be-tested object extraction apparatus according to claim 3, wherein the sample adding portion (31) further comprises a consumable storage bin (312) configured to carry a pipette tip, wherein the pipetting needle (311) is able to move to above the consumable storage bin (312) to load the pipette tip.

8. The to-be-tested object extraction apparatus according to claim 6, wherein the grabbing area (40) is further provided with a film sealing mechanism (43) configured to encapsulate the second reaction vessel, the reaction vessel transfer piece is further provided with a second transfer position located in the grabbing area (40), and when the reaction vessel transfer piece is located in the second transfer position, the reaction vessel gripper (411) is able to move to above the reaction vessel transfer piece to grab the second reaction vessel loaded with the nucleic acid and move to above the film sealing mechanism (43) to release the second reaction vessel to the film sealing mechanism (43).

9. The to-be-tested object extraction apparatus according to claim 8, wherein the film sealing mechanism (43) comprises a hot pressing piece (431) and a film sealing mechanism transfer piece (432), wherein the film sealing mechanism transfer piece (432) is movably arranged below the hot pressing piece (431) in the second horizontal direction, the film sealing mechanism transfer piece (432) is provided with a cover body carrying part (433) configured to place a cover body and a second reaction vessel carrying part (434) configured to place the second reaction vessel, the reaction vessel gripper (411) is able to release the second reaction vessel to the second reaction vessel carrying part (434), the reaction vessel gripper (411) is provided with a grabbing position located above the film sealing mechanism transfer piece (432), and when the reaction vessel gripper (411) is located in the grabbing position, the film sealing mechanism transfer piece (432) is provided with a separation position where the cover body carrying part (433) moves to below the reaction vessel gripper (411), so that the reaction vessel gripper (411) grabs the cover body, a release position where the second reaction vessel carrying part (434) moves to below the reaction vessel gripper (411), so that the reaction vessel gripper (411) releases the cover body to the second reaction vessel and an encapsulation position where the second reaction vessel carrying part (434) moves to below the hot pressing piece (431).

10. The to-be-tested object extraction apparatus according to claim 6, wherein the grabbing area (40) is further provided with a waste liquid station (42) located below the reaction vessel gripper (411), the waste liquid station (42) comprises a waste liquid rack (421), a pallet (422), and a waste liquid recycling piece (423), wherein the liquid waste recycling piece (423) is movably arranged on the liquid waste rack (421) in the vertical direction and the second horizontal direction, when the reaction vessel transfer piece is located in the first transfer position, the reaction vessel gripper (411) is able to move to above the first reaction vessel transfer piece (60) to grab the first reaction vessel and move to above the pallet (422) to release the first reaction vessel to the pallet (422), and the waste liquid recycling piece (423) is able to move to above the pallet (422) to recycle a magnetic rod sleeve and waste liquid.

11. The to-be-tested object extraction apparatus according to claim 10, wherein the waste liquid station (42) further comprises a support plate (424), a push rod (425), and a recycling bin, wherein the recycling bin is located below the waste liquid rack (421), the pallet (422) is vertically movably arranged on the waste liquid rack (421), the push rod (425) is movably arranged on the support plate (424) in the second horizontal direction, the support plate (424) is provided with an avoidance opening configured to avoid the pallet (422), the pallet (422) is able to move to below the support plate (424) to remain the first reaction vessel on the support plate (424), and the push rod (425) is able to push the first reaction vessel to recycle the first reaction vessel to the recycling bin.

12. A nucleic acid testing integrated machine, comprising a reagent preparation apparatus, a to-be-tested object extraction apparatus, and an amplification apparatus, wherein the reagent preparation apparatus is configured to prepare an extraction reagent and an amplification reagent, the amplification apparatus is configured to perform analyte detection determination on a determination mixture to analyze a to-be-tested object, and the to-be-tested object extraction apparatus is the to-be-tested object extraction apparatus according to any one of claims 1-11.

13. The nucleic acid testing integrated machine according to claim 12, wherein,
the reagent preparation apparatus, the to-be-tested object extraction apparatus, and the amplification apparatus are isolated from each other, the reagent preparation apparatus and the amplification apparatus are respectively located on both sides of the to-be-tested object extraction apparatus, a first channel is arranged between the reagent preparation apparatus and the to-be-tested object extraction apparatus, a second channel is arranged between the to-be-tested object extraction apparatus and the amplification apparatus, and the first channel and the second channel have an on state and an off state; and/or,
the nucleic acid testing integrated machine further comprises a ferry mechanism arranged between the reagent preparation device and the to-be-tested object extraction apparatus, wherein the ferry mechanism is able to move a first reaction vessel configured to prepare the extraction reagent and a second reaction vessel configured to prepare the amplification reagent to the to-be-tested object extraction apparatus through the first channel, and a grabbing portion (41) of the to-be-tested object extraction apparatus is able to move the first reaction vessel to a first reaction vessel placement position of the to-be-tested object extraction apparatus and move the second reaction vessel to a second reaction vessel placement position of the to-be-tested object extraction apparatus.

## Patentansprüche

1. Extraktionsvorrichtung für zu prüfende Objekte, umfassend:
einen Ladebereich (10) für zu prüfende Objekte, der mit einem Ladeabschnitt (11) für zu prüfende Objekte bereitgestellt ist, wobei der Ladeabschnitt für zu prüfende Objekte (11) so konfiguriert ist, dass er ein zu prüfendes Objektrohr aufnehmen und bearbeiten kann; wobei der Ladeabschnitt (11) für zu prüfende Objekte einen Behälter (112) für zu prüfende Objekte umfasst, der zur Aufnahme des zu prüfenden Objektrohrs konfiguriert ist;
einen Probenreaktionsbereich (20), der mit einem Probenreaktionsabschnitt (21) bereitgestellt ist, wobei der Probenreaktionsabschnitt (21) konfiguriert ist, um Nukleinsäure aus einem zu prüfenden Objekt zu extrahieren; der Probenreaktionsabschnitt (21) umfasst einen Extraktionsmechanismus;
einen Probenzugabebereich (30), der mit einem Probenzugabeabschnitt (31) bereitgestellt ist, wobei der Probenzugabeabschnitt (31) konfiguriert ist, sodass er das zu untersuchende Objekt vom Ladeabschnitt (11) für das zu untersuchende Objekt in ein erstes Reaktionsgefäß überführt und eine im Probenreaktionsabschnitt (21) extrahierte Nukleinsäure in ein zweites Reaktionsgefäß einbringt;
ein Rohrtransferstück (50) für zu prüfende Objekte, das beweglich zwischen dem Ladebereich (10) für zu prüfende Objekte und dem Probenzugabebereich (30) angeordnet ist, um das Probenröhrchen vom Ladebereich (10) für zu prüfende Objekte zum Probenzugabebereich (30) zu befördern, wobei das Rohrtransferstück (50) für zu prüfende Objekte mit einer ersten Arbeitsposition bereitgestellt ist, die sich im Ladebereich (10) für zu prüfende Objekte befindet; und
ein Greifbereich (40), der mit einem Greifabschnitt (41) bereitgestellt ist;
wobei der Ladebereich (10) für zu prüfende Objekte, der Bereich (30) zum Hinzufügen der Probe und der Greifbereich (40) um den Probenreaktionsbereich (20) herum angeordnet sind und sich der Greifbereich (40) auf derselben Seite wie der Ladebereich (10) für zu prüfende Objekte, der Probenreaktionsbereich (20) und dem Bereich für die Abtastung (30)
**dadurch gekennzeichnet, dass**
die Extraktionsvorrichtung für zu prüfende Objekte ferner ein Reaktionsgefäß-Transferstück und ein Extraktionsmechanismus-Transferstück (80) umfasst; wobei das Reaktionsgefäß-Transferstück beweglich zwischen dem Probenzugabebereich (30) und dem Greifbereich (40) angeordnet ist, um das erste Reaktionsgefäß und das zweite Reaktionsgefäß im Probenzugabebereich (30) zwischen dem Probenzugabebereich (30) und dem Greifbereich (40) zu bewegen, und das Transportstück (80) für den Extraktionsmechanismus beweglich zwischen dem Probenreaktionsbereich (20) und dem Greifbereich (40) angeordnet ist, um das erste Reaktionsgefäß zwischen dem Probenreaktionsbereich (20) und dem Greifbereich (40) zu bewegen;
der Greifabschnitt (41) konfiguriert ist, um das erste Reaktionsgefäß nach dem Zugabe des zu prüfenden Objekts vom Reaktionsgefäß-Transferstück zum Extraktionsmechanismus-Transferstück (80) zu bewegen und das erste Reaktionsgefäß nach der Extraktion der Nukleinsäure aus dem zu prüfenden Objekt vom Extraktionsmechanismus-Transferstück (80) zum Reaktionsgefäß-Transferstück zu bewegen;
und der Ladeabschnitt (11) für zu prüfende Objekte ferner einen Greifer (111) für zu prüfende Objektrohre umfasst, wobei der Greifer (111) für zu prüfende Objektrohre beweglich über dem Behälter (112) für zu prüfende Objekte und dem Transferstück (50) für zu prüfende Objektrohre angeordnet ist, und wenn sich das Transferstück (50) für zu prüfende Objektrohre in der ersten Position befindet, ist der Greifer (111) für zu prüfende Objektrohre in der Lage, das zu prüfende Objektrohr vom Behälter (112) für zu prüfende Objekte zum Transferstück (50) für zu prüfende Objektrohre zu bewegen.

2. Extraktionsvorrichtung für zu prüfende Objekte nach Anspruch 1, wobei das Transferstück (50) für zu prüfende Objektrohre in einer ersten horizontalen Richtung zwischen dem Ladebereich (10) für zu prüfende Objekte und dem Probenzugabebereich (30) beweglich angeordnet ist, das Reaktionsgefäß-Transferstück in einer zweiten horizontalen Richtung zwischen dem Probenzugabebereich (30) und dem Greifbereich (40) beweglich angeordnet ist, das Extraktionsmechanismus-Transferstück (80) in der zweiten horizontalen Richtung zwischen dem Probenreaktionsbereich (20) und dem Greifbereich (40) beweglich angeordnet ist, und die erste horizontale Richtung senkrecht zur zweiten horizontalen Richtung verläuft.

3. Extraktionsvorrichtung für zu prüfende Objekte nach Anspruch 2, wobei
das Reaktionsgefäß-Transferstück ein erstes Reaktionsgefäß-Transferstück (60) und ein zweites Reaktionsgefäß-Transferstück (70) umfasst, die parallel angeordnet sind, wobei das erste Reaktionsgefäß-Transferstück (60) und das zweite Reaktionsgefäß-Transferstück (70) in der zweiten horizontalen Richtung beweglich zwischen dem Probenzugabebereich und dem Greifbereich angeordnet sind; der Probenzugabeabschnitt (31) eine Pipettiernadel (311) umfasst, wobei die Pipettiernadel (311) beweglich oberhalb des ersten Reaktionsgefäß-Transferstücks (60) und des zweiten Reaktionsgefäß-Transferstücks (70) angeordnet ist, das erste Reaktionsgefäß-Transferstück (60) mit einer Probenzugabeposition bereitgestellt ist, die in dem Probenzugabebereich (30) angeordnet ist, und wenn sich das erste Reaktionsgefäß-Transferstück (60) in der Probenzugabeposition befindet, ist die Pipettiernadel (311) in der Lage, sich über das erste Reaktionsgefäß-Transferstück (60) zu bewegen, um das zu prüfende Objekt in das erste Reaktionsgefäß zu füllen.

4. Extraktionsvorrichtung für zu prüfende Objekte nach Anspruch 3, wobei
das zweite Reaktionsgefäß-Transferstück (70) mit einer Nukleinsäure-Zugabeposition bereitgestellt ist, die sich in dem Probenzugabebereich (30) befindet und mit dem ersten Reaktionsgefäß-Transferstück (60) bündig ist, und wenn das erste Reaktionsgefäß-Transferstück (60) sich in der Probenzugabeposition befindet und das zweite Reaktionsgefäß-Transferstück (70) sich in der Nukleinsäure-Zugabeposition befindet, die Pipettiernadel (311) in der Lage ist, sich oberhalb des ersten Reaktionsgefäß-Transferstücks (60) zu bewegen, um die extrahierte Nukleinsäure anzusaugen und sich oberhalb des zweiten Reaktionsgefäß-Transferstücks (70) zu bewegen, um die extrahierte Nukleinsäure in das zweite Reaktionsgefäß zu übertragen.

5. Extraktionsvorrichtung für zu prüfende Objekte nach Anspruch 3, wobei das Transferstück (50) für zu prüfende Objektrohre mit einer zweiten Arbeitsposition bereitgestellt ist, die sich in dem Probenzugabebereich (30) befindet, und wenn sich das Transferstück (50) für zu prüfende Objektrohre in der zweiten Arbeitsposition befindet, ist die Pipettiernadel (311) in der Lage, sich über das Transferstück (50) für zu prüfende Objektrohre zu bewegen, um das zu prüfende Objekt anzusaugen.

6. Extraktionsvorrichtung für zu prüfende Objekte nach Anspruch 3, wobei
der Greifabschnitt (41) einen Reaktionsgefäßgreifer (411) umfasst, der in der ersten horizontalen Richtung und einer vertikalen Richtung beweglich angeordnet ist, wobei das Extraktionsmechanismus-Transferstück (80) mit einer Aufnahmeposition, die in dem Greifbereich (40) angeordnet ist, und einer Extraktionsposition, die in dem Probenreaktionsbereich (20) angeordnet ist, versehen ist, das Reaktionsgefäß-Transferstück mit einer ersten Transferposition, die in dem Greifbereich (40) angeordnet ist, versehen ist und wenn das Reaktionsgefäß-Transferstück in der ersten Transferposition angeordnet ist und das Extraktionsmechanismus-Transferstück (80) in der Aufnahmeposition angeordnet ist, ist der Reaktionsgefäß-Greifer (411) in der Lage, das erste Reaktionsgefäß zu greifen, um das erste Reaktionsgefäß zwischen dem Reaktionsgefäß-Transferstück und dem Extraktionsmechanismus-Transferstück (80) zu bewegen.

7. Extraktionsvorrichtung für zu prüfende Objekte nach Anspruch 3, wobei der Probenzugabeabschnitt (31) ferner einen Vorratsbehälter (312) für Verbrauchsmaterial umfasst, der so konfiguriert ist, dass er eine Pipettenspitze trägt, wobei die Pipettiernadel (311) in der Lage ist, sich über den Vorratsbehälter (312) für Verbrauchsmaterial zu bewegen, um die Pipettenspitze zu laden.

8. Extraktionsvorrichtung für zu prüfende Objekte nach Anspruch 6, wobei der Greifbereich (40) ferner mit einem Filmversiegelungsmechanismus (43) bereitgestellt ist, der so konfiguriert ist, dass er das zweite Reaktionsgefäß einkapselt, wobei das Reaktionsgefäß-Transferstück ferner mit einer zweiten Transferposition versehen ist, die sich in dem Greifbereich (40) befindet, und wenn sich das Reaktionsgefäß-Transferstück in der zweiten Transferposition befindet, ist der Reaktionsgefäß-Greifer (411) in der Lage, sich oberhalb des Reaktionsgefäß-Transferstücks zu bewegen, um das mit der Nukleinsäure beladene zweite Reaktionsgefäß zu ergreifen, und sich oberhalb des Filmversiegelungsmechanismus (43) zu bewegen, um das zweite Reaktionsgefäß an den Filmversiegelungsmechanismus (43) freizugeben.

9. Extraktionsvorrichtung für zu prüfende Objekte nach Anspruch 8, wobei der Filmversiegelungsmechanismus (43) ein Heißpressstück (431) und ein Filmversiegelungsmechanismus-Transferstück (432) umfasst, wobei das Filmversiegelungsmechanismus-Transferstück (432) in der zweiten horizontalen Richtung beweglich unter dem Heißpressstück (431) angeordnet ist, das Filmversiegelungsmechanismus-Transferstück (432) mit einem Abdeckkörper-Tragteil (433), das konfiguriert ist, um einen Abdeckkörper zu platzieren, und einem zweiten Reaktionsgefäß-Tragteil (434), das konfiguriert ist, um das zweite Reaktionsgefäß zu platzieren, bereitgestellt ist, der Reaktionsgefäß-Greifer (411) in der Lage ist, das zweite Reaktionsgefäß an das zweite Reaktionsgefäß-Tragteil (434) freizugeben, der Reaktionsgefäß-Greifer (411) mit einer Greifposition bereitgestellt ist, die sich oberhalb des Filmversiegelungsmechanismus-Transferstücks (432) befindet, und wenn sich der Reaktionsgefäßgreifer (411) in der Greifposition befindet, das Filmversiegelungsmechanismus-Transferstück (432) mit einer Trennposition bereitgestellt ist, in der sich das Abdeckkörper-Tragteil (433) unter den Reaktionsgefäßgreifer (411) bewegt, so dass der Reaktionsgefäßgreifer (411) den Abdeckkörper ergreift, eine Freigabeposition, in der sich das zweite Reaktionsgefäß tragende Teil (434) unter den Reaktionsgefäßgreifer (411) bewegt, so dass der Reaktionsgefäßgreifer (411) den Abdeckkörper an das zweite Reaktionsgefäß freigibt, und eine Einkapselungsposition, in der sich das zweite Reaktionsgefäß-Tragteil (434) unter das Heißpressstück (431) bewegt.

10. Extraktionsvorrichtung für zu prüfende Objekte nach Anspruch 6, wobei der Greifbereich (40) ferner mit einer Abfallflüssigkeitsstation (42) bereitgestellt ist, die unterhalb des Reaktionsgefäßgreifers (411) angeordnet ist, wobei die Abfallflüssigkeitsstation (42) ein Abfallflüssigkeitsregal (421), eine Palette (422) und ein Abfallflüssigkeits-Recyclingstück (423) umfasst, wobei das Abfallflüssigkeits-Recyclingstück (423) auf dem Abfallflüssigkeitsregal (421) in der vertikalen Richtung und der zweiten horizontalen Richtung beweglich angeordnet ist, wenn sich das Reaktionsgefäß-Transferstück in der ersten Transferposition befindet, der Reaktionsgefäß-Greifer (411) in der Lage ist, sich über das erste Reaktionsgefäß-Transferstück (60) zu bewegen, um das erste Reaktionsgefäß zu ergreifen und sich über die Palette (422) zu bewegen, um das erste Reaktionsgefäß auf die Palette (422) freizugeben, und das Abfallflüssigkeits-Recyclingstück (423) in der Lage ist, sich über die Palette (422) zu bewegen, um eine Magnetstabhülse und Abfallflüssigkeit zu recyceln.

11. Extraktionsvorrichtung für zu prüfende Objekte nach Anspruch 10, wobei die Abfallflüssigkeitsstation (42) ferner eine Stützplatte (424), eine Schubstange (425) und einen Recycling-Behälter umfasst, wobei der Recycling-Behälter unter dem Abfallflüssigkeitsregal (421) angeordnet ist, die Palette (422) vertikal beweglich auf dem Abfallflüssigkeitsregal (421) angeordnet ist, die Schubstange (425) beweglich auf der Stützplatte (424) in der zweiten horizontalen Richtung angeordnet ist, die Stützplatte (424) mit einer Ausweichöffnung bereitgestellt ist, die so konfiguriert ist, dass sie die Palette (422) ausweicht, die Palette (422) in der Lage ist, sich unter die Stützplatte (424) zu bewegen, um das erste Reaktionsgefäß auf der Stützplatte (424) zu halten, und die Schubstange (425) in der Lage ist, das erste Reaktionsgefäß zu schieben, um das erste Reaktionsgefäß in den Recyclingbehälter zu recyceln.

12. Integrierte Maschine zum Prüfen von Nukleinsäuren, umfassend eine Reagenzvorbereitungsvorrichtung, eine Extraktionsvorrichtung für zu prüfende Objekte und eine Amplifikationsvorrichtung, wobei die Reagenzvorbereitungsvorrichtung so konfiguriert ist, dass sie ein Extraktionsreagenz und ein Amplifikationsreagenz vorbereitet, die Amplifikationstestvorrichtung so konfiguriert ist, dass sie eine Analytnachweisbestimmung an einem Bestimmungsgemisch durchführt, um ein zu prüfendes Objekt zu analysieren, und die Vorrichtung zur Verarbeitung von zu prüfenden Objekten die Vorrichtung zur Verarbeitung von zu prüfenden Objekten nach einem der Ansprüche 1-11 ist.

13. Integrierte Maschine zum Prüfen von Nukleinsäuren nach Anspruch 12, wobei,
die Reagenzvorbereitungsvorrichtung, die Extraktionsvorrichtung für zu prüfende Objekte und die Amplifikationsvorrichtung voneinander isoliert sind, die Reagenzvorbereitungsvorrichtung und die Verstärkungsvorrichtung jeweils auf beiden Seiten der Extraktionsvorrichtung für zu prüfende Objekte angeordnet sind, ein erster Kanal zwischen der Reagenzvorbereitungsvorrichtung und der Extraktionsvorrichtung für zu prüfende Objekte angeordnet ist, ein zweiter Kanal zwischen der Extraktionsvorrichtung für zu prüfende Objekte und der Verstärkungsvorrichtung angeordnet ist und der erste Kanal und der zweite Kanal einen Einschaltzustand und einen Ausschaltzustand haben; und/oder,
die integrierte Maschine zum Prüfen von Nukleinsäuren ferner einen Fährmechanismus umfasst, der zwischen der Reagenzvorbereitungsvorrichtung und der Extraktionsvorrichtung für zu prüfende Objekte angeordnet ist, wobei der Fährmechanismus in der Lage ist, ein erstes Reaktionsgefäß, das konfiguriert ist, um das Extraktionsreagenz vorzubereiten, und ein zweites Reaktionsgefäß, das konfiguriert ist, um das Amplifikationsreagenz vorzubereiten, zu der Extraktionsvorrichtung für zu prüfende Objekte durch den ersten Kanal zu bewegen, und ein Greifabschnitt (41) der Extraktionsvorrichtung für zu prüfende Objekte in der Lage ist, das erste Reaktionsgefäß zu einer ersten Reaktionsgefäßplatzierungsposition der Extraktionsvorrichtung für zu prüfende Objekte zu bewegen und das zweite Reaktionsgefäß zu einer zweiten Reaktionsgefäßplatzierungsposition der Extraktionsvorrichtung für zu prüfende Objekte zu bewegen.

## Revendications

1. Appareil d'extraction d'objet à tester, comprenant :
une zone de chargement (10) d'objets à tester, dotée d'une partie de chargement (11) d'objets à tester, la partie de chargement (11) d'objets à tester étant configurée pour stocker et traiter un tube d'objet à tester ; la partie de chargement (11) d'objets à tester comprenant un bac (112) d'objets à tester configuré pour stocker le tube d'objet à tester ;
une zone de réaction (20) d'échantillon, dotée d'une partie de réaction (21) d'échantillon, la partie de réaction (21) d'échantillon étant configurée pour extraire l'acide nucléique d'un objet à tester ; la partie de réaction (21) d'échantillon comprenant un mécanisme d'extraction ;
une zone d'ajout (30) d'échantillon, dotée d'une partie d'ajout (31) d'échantillon, la partie d'ajout (31) d'échantillon étant configurée pour transférer l'objet à tester de la partie de chargement (11) d'objets à tester vers un premier récipient de réaction et ajouter un acide nucléique extrait dans la partie de réaction (21) d'échantillon à un second récipient de réaction ;
une pièce de transfert de tube (50) d'objet à tester, qui est disposée de manière mobile entre la zone de chargement (10) d'objets à tester et la zone d'ajout (30) d'échantillon pour déplacer le tube d'objet à tester dans la zone de chargement (10) d'objets à tester vers la zone d'ajout (30) d'échantillon, la pièce de transfert de tube (50) d'objet à tester étant dotée d'une première position de travail située dans la zone de chargement (10) d'objets à tester ; et
une zone de préhension (40), pourvue d'une partie de préhension (41) ;
la zone de chargement (10) d'objets à tester, la zone d'ajout (30) d'échantillon et la zone de préhension (40) étant disposées autour d'un extérieur de la zone de réaction (20) d'échantillon, et la zone de préhension (40) étant située du même côté de la zone de chargement (10) d'objets à tester, de la zone de réaction (20) d'échantillon et de la zone d'ajout (30) d'échantillon ;
**caractérisé en ce que**
l'appareil d'extraction d'objet à tester comprend en outre une pièce de transfert de récipient de réaction et une pièce de transfert (80) de mécanisme d'extraction ; la pièce de transfert de récipient de réaction étant disposée de manière mobile entre la zone d'ajout (30) d'échantillon et la zone de préhension (40) pour déplacer le premier récipient de réaction et le second récipient de réaction dans la zone d'ajout (30) d'échantillon entre la zone d'ajout (30) d'échantillon et la zone de préhension (40), et la pièce de transfert (80) de mécanisme d'extraction est disposée de manière mobile entre la zone de réaction (20) d'échantillon et la zone de préhension (40) pour déplacer le premier récipient de réaction entre la zone de réaction (20) d'échantillon et la zone de préhension (40) ;
la partie de préhension (41) est configurée pour déplacer le premier récipient de réaction après avoir ajouté l'objet à tester de la pièce de transfert de récipient de réaction à la pièce de transfert (80) de mécanisme d'extraction et pour déplacer le premier récipient de réaction après avoir extrait l'acide nucléique de l'objet à tester de la pièce de transfert (80) de mécanisme d'extraction à la pièce de transfert de récipient de réaction ;
et la partie de chargement (11) d'objets à tester comprend en outre un préhenseur de tube (111) d'objet à tester, le préhenseur de tube (111) d'objet à tester étant disposé de manière mobile au-dessus d'un bac (112) d'objets à tester et de la pièce de transfert de tube (50) d'objet à tester, et lorsque la pièce de transfert de tube (50) d'objet à tester est située dans la première position de travail, le préhenseur de tube (111) d'objet à tester est capable de déplacer le tube d'objet à tester du bac (112) d'objets à tester à la pièce de transfert de tube (50) d'objet à tester.

2. Appareil d'extraction d'objet à tester selon la revendication 1, la pièce de transfert de tube (50) d'objet à tester étant disposée de manière mobile entre la zone de chargement (10) d'objets à tester et la zone d'ajout (30) d'échantillon dans une première direction horizontale, la pièce de transfert de récipient de réaction étant disposée de manière mobile entre la zone d'ajout (30) d'échantillon et la zone de préhension (40) dans une seconde direction horizontale, la pièce de transfert (80) de mécanisme d'extraction étant disposée de manière mobile entre la zone de réaction (20) d'échantillon et la zone de préhension (40) dans la seconde direction horizontale, et la première direction horizontale étant perpendiculaire à la seconde direction horizontale.

3. Appareil d'extraction d'objet à tester selon la revendication 2, dans lequel,
la pièce de transfert de récipient de réaction comprend une première pièce de transfert (60) de récipient de réaction et une seconde pièce de transfert (70) de récipient de réaction qui sont disposées en parallèle, la première pièce de transfert (60) de récipient de réaction et la seconde pièce de transfert (70) de récipient de réaction étant disposées de manière mobile entre la zone d'ajout d'échantillon et la zone de préhension dans la seconde direction horizontale ; la partie d'ajout (31) d'échantillon comprenant une aiguille de pipetage (311), l'aiguille de pipetage (311) étant disposée de manière mobile au-dessus de la première pièce de transfert (60) de récipient de réaction et de la seconde pièce de transfert (70) de récipient de réaction, la première pièce de transfert (60) de récipient de réaction étant pourvue d'une position d'ajout d'échantillon située dans la zone d'ajout (30) d'échantillon, et lorsque la première pièce de transfert (60) de récipient de réaction est située dans la position d'ajout d'échantillon, l'aiguille de pipetage (311) peut se déplacer au-dessus de la première pièce de transfert (60) de récipient de réaction pour remplir l'objet à tester dans le premier récipient de réaction.

4. Appareil d'extraction d'objet à tester selon la revendication 3, dans lequel,
la seconde pièce de transfert (70) de récipient de réaction est pourvue d'une position d'ajout d'acide nucléique située dans la zone d'ajout (30) d'échantillon et au même niveau de la première pièce de transfert (60) de récipient de réaction, et lorsque la première pièce de transfert (60) de récipient de réaction est située dans la position d'ajout d'échantillon et que la seconde pièce de transfert (70) de récipient de réaction est située dans la position d'ajout d'acide nucléique, l'aiguille de pipetage (311) peut se déplacer au-dessus de la première pièce de transfert (60) de récipient de réaction pour aspirer l'acide nucléique extrait et se déplacer au-dessus de la seconde pièce de transfert (70) de récipient de réaction pour transférer l'acide nucléique extrait au second récipient de réaction.

5. Appareil d'extraction d'objet à tester selon la revendication 3, la pièce de transfert de tube (50) d'objet à tester étant dotée d'une seconde position de travail située dans la zone d'ajout (30) d'échantillon, et lorsque la pièce de transfert de tube (50) d'objet à tester est située dans la seconde position de travail, l'aiguille de pipetage (311) peut se déplacer au-dessus de la pièce de transfert de tube (50) d'objet à tester afin d'aspirer l'objet à tester.

6. Appareil d'extraction d'objet à tester selon la revendication 3, dans lequel,
la partie de préhension (41) comprend un préhenseur (411) de récipient de réaction disposé de manière mobile dans la première direction horizontale et dans une direction verticale, la pièce de transfert (80) de mécanisme d'extraction ayant une position de réception située dans la zone de préhension (40) et une position d'extraction située dans la zone de réaction (20) d'échantillon, la pièce de transfert de récipient de réaction est pourvue d'une première position de transfert située dans la zone de préhension (40), et lorsque la pièce de transfert de récipient de réaction se trouve dans la première position de transfert et que la pièce de transfert (80) de mécanisme d'extraction se trouve dans la position de réception, le préhenseur (411) de récipient de réaction peut saisir le premier récipient de réaction pour déplacer le premier récipient de réaction entre la pièce de transfert de récipient de réaction et la pièce de transfert (80) de mécanisme d'extraction.

7. Appareil d'extraction d'objet à tester selon la revendication 3, la partie d'ajout (31) d'échantillon comprenant en outre un bac de stockage (312) de consommables configuré pour transporter une pointe de pipette, l'aiguille de pipetage (311) étant capable de se déplacer au-dessus du bac de stockage (312) de consommables pour charger la pointe de pipette.

8. Appareil d'extraction d'objet à tester selon la revendication 6, la zone de préhension (40) étant en outre pourvue d'un mécanisme de scellage de film (43) configuré pour encapsuler le second récipient de réaction, la pièce de transfert de récipient de réaction étant en outre pourvue d'une seconde position de transfert située dans la zone de préhension (40), et lorsque la pièce de transfert de récipient de réaction est située dans la seconde position de transfert, le préhenseur (411) de récipient de réaction peut se déplacer au-dessus de la pièce de transfert de récipient de réaction pour saisir le second récipient de réaction chargé de l'acide nucléique et se déplacer au-dessus du mécanisme de scellage de film (43) pour libérer le second récipient de réaction vers le mécanisme de scellage de film (43).

9. Appareil d'extraction d'objet à tester selon la revendication 8, le mécanisme de scellage de film (43) comprenant une pièce de pressage à chaud (431) et une pièce de transfert (432) de mécanisme de scellage de film, la pièce de transfert (432) de mécanisme de scellage de film étant disposée de manière mobile sous la pièce de pressage à chaud (431) dans la seconde direction horizontale, la pièce de transfert (432) de mécanisme de scellage de film étant pourvue d'une partie de transport (433) de corps de couvercle configurée pour placer un corps de couvercle et d'une seconde partie de transport (434) de récipient de réaction configurée pour placer le second récipient de réaction, le préhenseur (411) de récipient de réaction étant capable de libérer le second récipient de réaction sur la seconde partie de transport (434) de récipient de réaction, le préhenseur (411) de récipient de réaction est doté d'une position de préhension située au-dessus de la pièce de transfert (432) de mécanisme de scellage de film, et lorsque le préhenseur (411) de récipient de réaction est situé dans la position de préhension, la pièce de transfert (432) de mécanisme de scellage de film est dotée d'une position de séparation où la partie de transport (433) de corps de couvercle se déplace sous le préhenseur (411) de récipient de réaction, de sorte que le préhenseur (411) de récipient de réaction saisisse le corps de couvercle, une position de libération où la seconde partie de transport (434) de récipient de réaction se déplace sous le préhenseur (411) de récipient de réaction, de sorte que le préhenseur (411) de récipient de réaction libère le corps de couvercle au second récipient de réaction, et une position d'encapsulation où la seconde partie de transport (434) de récipient de réaction se déplace sous la pièce de pressage à chaud (431).

10. Appareil d'extraction d'objet à tester selon la revendication 6, la zone de préhension (40) étant en outre pourvue d'un poste (42) de liquide usagé situé sous le préhenseur (411) de récipient de réaction, le poste (42) de liquide usagé comprenant un support (421) de liquide usagé, une palette (422) et une pièce de recyclage (423) de liquide usagé, la pièce de recyclage (423) de liquide usagé étant disposée de manière mobile sur le support (421) de liquide usagé dans la direction verticale et dans la seconde direction horizontale, lorsque la pièce de transfert de récipient de réaction est située dans la première position de transfert, le préhenseur (411) de récipient de réaction peut se déplacer au-dessus de la première pièce de transfert (60) de récipient de réaction pour saisir le premier récipient de réaction et se déplacer au-dessus de la palette (422) pour libérer le premier récipient de réaction sur la palette (422), et la pièce de recyclage (423) de liquide usagé peut se déplacer au-dessus de la palette (422) pour recycler un manchon de tige magnétique et le liquide usagé.

11. Appareil d'extraction d'objet à tester selon la revendication 10, le poste (42) de liquide usagé comprenant en outre une plaque de support (424), une tige de poussée (425) et un bac de recyclage, le bac de recyclage étant situé sous le support (421) de liquide usagé, la palette (422) étant disposée verticalement de manière mobile sur le support (421) de liquide usagé, la tige de poussée (425) étant disposée de manière mobile sur la plaque de support (424) dans la seconde direction horizontale, la plaque de support (424) étant pourvue d'une ouverture d'évitement configurée pour éviter la palette (422), la palette (422) pouvant se déplacer sous la plaque de support (424) pour maintenir le premier récipient de réaction sur la plaque de support (424), et la tige de poussée (425) pouvant pousser le premier récipient de réaction pour recycler le premier récipient de réaction dans le bac de recyclage.

12. Machine intégrée de test d'acide nucléique, comprenant un appareil de préparation de réactifs, un appareil d'extraction d'objet à tester et un appareil d'amplification, l'appareil de préparation de réactifs étant configuré pour préparer un réactif d'extraction et un réactif d'amplification, l'appareil d'amplification étant configuré pour effectuer une détermination de détection d'analyte sur un mélange de détermination afin d'analyser un objet à tester, et l'appareil d'extraction d'objet à tester étant l'appareil d'extraction d'objet à tester selon l'une quelconque des revendications 1-11.

13. Machine intégrée de test d'acide nucléique selon la revendication 12, dans laquelle,
l'appareil de préparation de réactifs, l'appareil d'extraction d'objet à tester et l'appareil d'amplification sont isolés l'un de l'autre, l'appareil de préparation de réactifs et l'appareil d'amplification sont respectivement situés sur les deux côtés de l'appareil d'extraction d'objet à tester, un premier canal est disposé entre l'appareil de préparation de réactifs et l'appareil d'extraction d'objet à tester, un second canal est disposé entre l'appareil d'extraction d'objet à tester et l'appareil d'amplification, et le premier canal et le second canal ont un état de marche et un état d'arrêt ; et/ou,
la machine intégrée de test d'acide nucléique comprend en outre un mécanisme de transfert disposé entre le dispositif de préparation de réactifs et l'appareil d'extraction d'objet à tester, le mécanisme de transfert étant capable de déplacer un premier récipient de réaction configuré pour préparer le réactif d'extraction et un second récipient de réaction configuré pour préparer le réactif d'amplification vers l'appareil d'extraction d'objet à tester par l'intermédiaire du premier canal, et une partie de préhension (41) de l'appareil d'extraction d'objet à tester est capable de déplacer le premier récipient de réaction vers une première position de placement de récipient de réaction de l'appareil d'extraction d'objet à tester et de déplacer le second récipient de réaction vers une seconde position de placement de récipient de réaction de l'appareil d'extraction d'objet à tester.
